# EUROPEAN PATENT SPECIFICATION

(11) **EP 2 596 354 B1**
(45) Date of publication and mention of the grant of the patent: **12.04.2017**
(21) Application number: 11810481.9
(22) Date of filing: 22.07.2011
(51) Int. Cl.: G01N 33/53, G01N 33/543, G01N 33/533

(54) **CO-COUPLING TO CONTROL REACTIVITY OF REAGENTS IN IMMUNOASSAYS**
KO-KOPPLUNG ZUR KONTROLLE DER REAKTIVITÄT VON REAGENZIEN BEI IMMUNOASSAYS
CO-COUPLAGE POUR RÉGULER LA RÉACTIVITÉ DES RÉACTIFS DANS DES DOSAGES IMMUNOLOGIQUES

(30) Priority: 23.07.2010 US 367281 P
(43) Date of publication of application: 29.05.2013
(73) Proprietor: Luminex Corporation, Austin, TX 78727 (US)
(72) Inventor: BAKER, Harold, Austin, TX 78727 (US); HOFFMEYER, Michaela, Austin, TX 78727 (US); DUNBAR, Sherry, Austin, TX 78727 (US)
(74) Representative: D Young & Co LLP
(86) International application number: PCT/US2011/045053
(87) International publication number: WO 2012/012748

(56) References cited:
- EP-A1- 1 777 521
- WO-A1-2006/074076
- WO-A2-01/63284
- US-A- 4 357 311
- US-A- 4 812 414
- US-A- 5 143 825
- US-A- 5 861 262
- US-A1- 2002 182 146
- US-A1- 2006 211 061
- US-A1- 2009 226 890
- US-A1- 2009 226 939

## Description

### CROSS-REFERENCE TO RELATED APPLICATIONS

The present application claims benefit of priority to U.S. Provisional Application Serial No. 61/367,281 filed July 23, 2010.

### BACKGROUND OF THE INVENTION

### A. Field of the Invention

The present description relates generally to the field, of molecular biology. More particularly, it concerns methods and compositions relating to immunoassays. The present description also concerns co-coupling reagents to control reactivity in an immunoassay.

### B. Description of Related Art

It is desirable that the amount of signal (e.g., fluorescence, chemiluminescence, radioactivity) produced in an immunoassay fall within the usable range of the detection apparatus and/or generate an acceptable dose response curve for an immunoassay. In a singleplex assay, where the analysis is of one analyte, the amount of signal produced is typically adjusted by adjusting the amount of analyte in the assay. In multiplex assays, however, it may not be possible to appropriately adjust the amount of all analytes since increasing the amount of one analyte in order to raise its detectable signal above a minimum usable level may result in the detectable signal for another analyte being raised above the maximum usable level. Likewise, decreasing the amount of an analyte in order to lower its detectable level below a maximum usable level or achieve an acceptable dose response curve may result in the detectable signal for another analyte being lowered below the minimum usable level.

Methods for controlling immunoassay reactivity in multiplex assays often are focused on controlling reagents that are used in the later steps of an assay. This is commonly a "detection" antibody to which a biotin group or another functional group has been attached. The functional group may be an enzyme such as Horse Radish Peroxide or Alkaline Phosphatase as commonly used in ELISA and in other singleplex assays. Biotin or R-phycoerythrin are examples of other commonly used functional groups. Controlling reagents that are used in the later steps of an assay, however, risks providing unreliable information when concentrations of analytes are high. Concentrations of different analytes in the same biological sample can differ by more that 1,000 fold. For example, TSH is normally pglml while LH and FSH are ng/ml. As another example, when testing for Trisomy 23, Down's Syndrome, hCG, E3 and AFP are measured. HCG is at mg/mL, E3 is at ng/mL and AFP is at pg/mL. This is an eight log difference in concentration and makes a multiplex challenging. In some cases, there can be more than a 1,000-fold difference for the same analyte across different samples (e.g., hCG is normally ng/ml and increases to mg/ml during pregnancy) Accordingly, there is a need for methods and compositions that can provide controlled reactivity and balanced measurements in the presence of the highest concentrations of analytes and in samples containing analytes at significantly different concentrations.

### SUMMARY OF THE INVENTION

Described herein is a method for preparing a substrate for an immunoassay comprising: (a) obtaining a composition comprising a reagent and a neutral material; and (b) applying the composition to a substrate under conditions suitable to couple or coat the reagent and the neutral material to the substrate. Also described herein is a substrate on which a reagent and a neutral material are coupled or coated. The reagent and neutral material may be covalently coupled to the substrate. Further described herein is a kit comprising a substrate, a reagent, and a neutral material. The substrate, reagent, and neutral material may be packaged separately in the kit, or the reagent and neutral material may be provided together in a composition, or the reagent and neutral material may be provided coupled or coated on the substrate.

Described herein is a method of controlling reactivity of a reagent in a multiplex immunoassay comprising: (a) combining a high-reactivity reagent with a neutral material in a composition; and (b) applying the composition to a substrate under conditions suitable to couple the high-reactivity reagent and the neutral material to the substrate. The method may further comprises confirming that the assay signal of the high-reactivity reagent co-coupled with the neutral material to the substrate is within a usable signal range for the multiplex immunoassay.

The present invention provides a method of controlling reactivity of a reagent in a multiplex immunoassay comprising:
(a) identifying a reagent having a high reactivity in a multiplex immunoassay resulting in an assay signal that is above a maximum usable signal for the multiplex immunoassay;
(b) combining the high-reactivity reagent with a neutral material in a composition, wherein the neutral material is a material that is antigenically neutral with regard to the reagent and the sample in the assay;
(c) applying the composition to a substrate under conditions suitable to couple the high-reactivity reagent and the neutral material to the substrate; and
(d) confirming that the assay signal of the high-reactivity reagent co-coupled with the neutral material to the substrate is within a usable signal range for the multiplex immunoassay.

In certain embodiments of the invention, the method comprises controlling the reactivity of two or more reagents having high reactivity in the multiplex immunoassay. The multiplex assay may include 2, 3, 4, 5, 6, 7, 8, 9, 10, 20, 50, 100, 200, 500, 1000, 2000 or more reagents of which a subset of these reagents have high reactivity in the multiplex immunoassay. The multiplex assay may include 2, 3, 4, 5, 6, 7, 8, 9, 10,20, 50, 100, 200, 500, 1000, 2000, 5000, or any range derivable therein, reagents of which a subset of these reagents have high reactivity in the multiplex immunoassay. The method may comprise controlling the reactivity of 3, 4, 5, 6, 7, 8, 9, 10, 20, 50, 100 or more reagents having high reactivity in the multiplex immunoassay. The method may comprise controlling the reactivity of 2, 3, 4, 5, 6, 7, 8, 9, 10, 20, 50, 100, 200, 500, 1000, 2000, 5000 or any range derivable therein, reagents having high reactivity in the multiplex immunoassay. A variety of detection techniques are known in the art and may be used to produce the assay signal. In some embodiments, the assay signal is a chemiluminescent signal or a fluorescent signal. The signal may be colorimetric signal or a radioactive signal. The usable range refers to the set of values for which the error lies within specified limits such that the results or signal is reliable.

As used herein, the phrase "multiplex" or grammatical equivalents refers to the parallel detection, analysis or amplification of more than one target analyte of interest per sample. Analysis of multiple different analytes (multiplex) may be performed simultaneously. Detection is performed on a variety of platforms, including but not limited to well plates and bead arrays.

The substrate may be any substrate that may be used in immunodetection methods. In some embodiments, the substrate is a particle or a surface of a well. The particle may be a microsphere. Non-limiting examples of such substrates include a well in a polystyrene microtiter plate or a microsphere. The substrate may be made of, for example, nitrocellulose, nylon membrane, glass, activated quartz, activated glass, silica, polyvinylidene difluoride (PVDF) membrane, polystyrene substrates, polyacrylamide-based substrate, other polymers, copolymers, or crosslinked polymers such as poly(vinyl chloride), poly(methyl methacrylate), poly(dimethyl siloxane), photopolymers (which contain photoreactive species such as nitrenes, carbenes and ketyl radicals capable of forming covalent links with target molecules). Molecules immobilized on planar solid supports are typically identified by their spatial position on the support. Molecules immobilized on non-planar solid supports, such as microspheres ("beads"), are often identified by some form of encoding of the support, as discussed below.

Beads may be encoded such that one subpopulation of beads can be distinguished from another subpopulation. Encoding may be by a variety of techniques. For example, the beads may be fluorescently labeled with fluorescent dyes having different emission spectra and/or different signal intensities. The beads may be Luminex MagPlex® Microspheres, Luminex xTAG® Microspheres, Luminex SeroMap™ Microspheres, or Luminex MicroPlex® Microspheres. Another encoding technology uses holographic coded or barcoded beads. The size of the beads in a subpopulation may also be used to distinguish one subpopulation from another. Another method of modifying a bead is to incorporate a magnetically responsive substance, such as Fe₃O₄, into the structure. Paramagnetic and superparamagnetic microspheres have negligible magnetism in the absence of a magnetic field, but application of a magnetic field induces alignment of the magnetic domains in the microspheres, resulting in attraction of the microspheres to the field source. Combining fluorescent dyes, bead size, and/or magnetically responsive substances into the beads can further increase the number of different subpopulations of beads that can be created.

A reagent refers to a substance used in testing for or reacting with other substances. The substance with which the reagent reacts may be referred to as the analyte. The analyte may be any substance to be detected and/or quantified. The analyte may be present in a sample, such as a bodily fluid (including but not limited to whole blood, serum, saliva, urine, sperm) or an environmental sample (including but not limited to water or soil).
The analyte may be an antigen or an antibody. The analyte may be, for example, a protein, lipid, carbohydrate, or nucleic acid. The reagent may be an antigen, an antibody, an aptamer, or oligonucleotide. In some embodiments, the high-reactivity reagent is an antibody or an antigen. The reagent may be a protein to which a small molecule target has been attached that can then be used in an immunoassay to measure the small molecule. For example, steroids, small molecule hormones, or other small molecules may be attached to BSA, and coupled to the substrate. Examples of these would be progestin molecules, estrogen molecules, and thyroid hormone molecules. These proteins, most commonly BSA to which small molecules have been coupled, can themselves be coupled to microspheres as is or can be co-coupled with a neutral material as in BSA to which many of these molecules have not been coupled.

As used herein, the term "antibody" is intended to refer broadly to any immunologic binding agent, such as IgY, IgG, IgM, IgA, IgD and IgE, and antigen-binding fragments thereof. In some embodiments, the antibody may be, for example, an antibody to *Haemophilus influenza* type b (Hib) polysaccharide and the toxoids of *Clostridium tetani* (Tet) and *Corynebacterium diphtheriae* (Dip), Streptococcus pneumoniae, Meningoccus, Polio, Diptheria, Tetanus, HIV, HBV, HCV.

The reagent may be a rabbit polyclonal antibody developed to recognize Chicken IgY. The rabbit anti-Chicken antibody may have biotin coupled to it to enable it to react with a Streptavidin molecule coupled to a detection regent, R-phycoerythrin. The rabbit polyclonal antibody may be coupled to microspheres.

The reagent may be a Mouse Monoclonal antibody developed to recognize Thyroid Stimulating Hormone (TSH).

The reagent or analyte may be a protein. The protein may be, for example, IgY, insulin, TSH, tetanus toxin or toxoid, diphtheria toxin or toxoid, pituitary hormones, trypsin or trypsinogen. The IgY may be reactive toward a disease causing organism (e.g., virus or bacteria) that has infected, or is suspected of having infected, the animal. The infected animal may be, for example, a vertebrate including mammals, rodents, and birds. The animal may be a human. The analyte may be an antibody to an infection, an infectious agent (e.g., viruses, bacteria, fungus), or a prion.

The neutral material is a material that is antigenically neutral with regard to the reagent and the sample in the assay. The neutral material may be a nonspecific protein such as serum albumin (e.g., bovine serum albumin (BSA)), casein, or a non-relevant species antibody. In some embodiments, the neutral material is a non-relevant species antibody or a serum albumin. Because the reagent and the neutral material are combined prior to coupling or coating them to a substrata, the reagent and the neutral material are co-coupled or co-coated to the same substrate or region of the substrate. For example, where the substrate is a bead, a mixture of reagent and neutral material is coupled to the same bead. Where the substrate is a well, a mixture of reagent and neutral material is coupled or coated on the surface of the same well. Following the initial coupling or coating of the reagent/neutral material mixture, additional neutral material may be added to "coat" any remaining available surfaces of the substrate. The coating allows for blocking of nonspecific adsorption sites on the immobilizing surface and thus reduces the background caused by nonspecific binding. In some embodiments, the high reactivity reagent and the neutral material are covalently coupled to the substrate.

A sample may contain varied concentrations of different analytes of interest. It can, therefore, be a challenge in a multiplex assay to achieve an assay signal from each analyte that is within the usable signal range for the assay. For example, if the assay signal for a first analyte exceeds the maximum usable signal for the assay, it may not be possible to correct this by diluting the sample because that would result in the assay signal for a second analyte being below the minimum usable signal for the assay. The methods and compositions disclosed herein address this challenge at the reagent side of the assay by co-coupling a reagent and a neutral material. The ratio of reagent to neutral material in a composition used for coupling may be adjusted as needed to achieve the desired assay signal.

The ratio of reagent to neutral material in the composition may be about 1:1,000, 1:500, 1:200, 1:100, 1:120, 1:100, 1:80, 1:60, 1:40, 1:20, 1:10, 1:5, 1:2, 1:1, 2:1, 4:1, 6:1, 8:1, 10:1, or 20:1, or any range derivable therein. For example, the range may be between about 1:120 to 6:1, 1:120 to 1:60, or 1:60 to 6:1. The ratio of reagent to neutral material coupled to a substrate may be adjusted as needed to achieve the desired assay signal.

The ratio of reagent to neutral material coupled to the substrate may be about 1:1,000, 1:500, 1:200, 1:100, 1:120, 1:100, 1:80, 1:60, 1:40, 1:20, 1:10, 1:5, 1:2,1:1,2:1,4:1, 6:1, 8:1, 10:1, or 20:1, or any range derivable therein. For example, the range may be between about 1:120 to 6:1,1:120 to 1:60, or 1:60 to 6:1. In some embodiments, the ratio of high-reactivity reagent to the neutral material is between about 1:120 to 6:1.

It is contemplated that any method or composition described herein can be implemented with respect to any other method or composition described herein.

The use of the term "or" in the claims is used to mean "and/or" unless explicitly indicated to refer to alternatives only or the alternatives are mutually exclusive, although the disclosure supports a definition that refers to only alternatives and "and/or."

Throughout this application, the term "about" is used to indicate that a value includes the standard deviation of error for the device or method being employed to determine the value.

Following long-standing patent law, the words "a" and "an," when used in conjunction with the word "comprising" in the claims or specification, denotes one or more, unless specifically noted.

The terms "comprise" (and any form of comprise, such as "comprises" and "comprising"), "have" (and any form of have, such as "has" and "having"), "contain" (and any form of contain, such as "contains" and "containing"), and "include" (and any form of include, such as "includes" and "including") are open-ended linking verbs. As a result, a method, composition, kit, or system that "comprises," "has," "contains," or "includes" one or more recited steps or elements possesses those recited steps or elements, but is not limited to possessing only those steps or elements; it may possess (i.e., cover) elements or steps that are not recited. Likewise, an element of a method, composition, kit, or system that "comprises," "has," "contains," or "includes" one or more recited features possesses those features, but is not limited to possessing only those features; it may possess features that are not recited.

Any embodiment of any of the present methods, composition, kit, and systems may consist of or consist essentially of-rather than comprise/include/contain/have-the described steps and/or features. Thus, in any of the claims, the term "consisting of" or "consisting essentially of" may be substituted for any of the open-ended linking verbs recited above, in order to change the scope of a given claim from what it would otherwise be using the open-ended linking verb.

Other objects, features and advantages of the present invention will become apparent from the following detailed description. It should be understood, however, that the detailed description and the specific examples, while indicating specific embodiments of the invention, are given by way of illustration only, since various changes and modifications within the scope of the invention will become apparent to those skilled in the art from this detailed description.

### DESCRIPTION OF ILLUSTRATIVE EMBODIMENTS

Embodiments of the present invention provide methods and compositions that control immunoassay reactivity at the beginning of the assay and thus provide a balanced control for all levels of measurement while controlling the reactivity at the highest concentrations of analytes. As discussed above, conventional methods for controlling immunoassay reactivity that focus on controlling reagents used in the later steps of an assay are susceptible to providing unreliable information when concentrations of analytes are high. Furthermore, while it is possible to simply couple less reagent to the solid phase material, the studies described below indicate that this approach results in non-uniformly coupled/coated surfaces that are less effective in controlling reactivity than the those prepared by the co-coupling or co-coating approaches disclosed herein, as these approaches provide both reactivity control and uniformity to the couple/coated surfaces.

### I. Immunoassays

The methods and compositions described herein may be used to control reactivity of reagents in immunoassays. One of skill in the art would understand that various methods exist for performing immunoassays, including but not limited to those described below.

Some immunodetection methods include enzyme linked immunosorbent assay (ELISA), radioimmunoassay (RIA), immunoradiometric assay, fluoroimmunoassay, chemiluminescent assay, bioluminescent assay, and Western blot to mention a few. The steps of various useful immunodetection methods have been described in the scientific literature, such as, *e.g.*, Doolittle and Ben-Zeev, 1999; Gulbis and Galand, 1993; De *Jager et al.*, 1993; and Nakamura *et al.*, 1987.

Contacting the chosen biological sample with the antibody under effective conditions and for a period of time sufficient to allow the formation of immune complexes (primary immune complexes) is generally a matter of simply adding the antibody composition to the sample and incubating the mixture for a period of time long enough for the antibodies to form immune complexes with, *i.e.*, to bind to, any antigens present. After this time, the sample-antibody composition, such as a tissue section, ELISA plate, dot blot or western blot, will generally be washed to remove any non-specifically bound antibody species, allowing only those antibodies specifically bound within the primary immune complexes to be detected.

In general, the detection of immunocomplex formation is well known in the art and may be achieved through the application of numerous approaches. These methods are generally based upon the detection of a label or marker, such as any of those radioactive, fluorescent, biological and enzymatic tags. Patents concerning the use of such labels include U.S. Patents 3,817,837, 3,850,752, 3,939,350, 3,996,345, 4,277,437, 4,275,149 and 4,366,241. Of course, one may find additional advantages through the use of a secondary binding ligand such as a second antibody and/or a biotin/avidin ligand binding arrangement, as is known in the art. In the presence of high concentrations of analytes, the amount of detectable signal produced by immunocomplex may exceed the maximum usable range for the detection apparatus. Accordingly, the present description provides methods and compositions that control immunoassay reactivity and thus can control the reactivity at the highest concentrations of analytes, whereby the detectable signal produced by immunocomplex falls within the usable signal range for the detection apparatus and acceptable ranges determined by regulatory authorities.

The selective antibody employed in the detection may itself be linked to a detectable label, wherein one would then simply detect this label, thereby allowing the amount of the primary immune complexes in the composition to be determined. Alternatively, the first antibody that becomes bound within the primary immune complexes may be detected by means of a second binding ligand that has binding affinity for the antibody. In these cases, the second binding ligand may be linked to a detectable label. The second binding ligand is itself often an antibody, which may thus be termed a "secondary" antibody. The primary immune complexes are contacted with the labeled, secondary binding ligand, or antibody, under effective conditions and for a period of time sufficient to allow the formation of secondary immune complexes. The secondary immune complexes are then generally washed to remove any non-specifically bound labeled secondary antibodies or ligands, and the remaining label in the secondary immune complexes is then detected.

Further methods include the detection of primary immune complexes by a two-step approach. A second binding ligand, such as an antibody, that has binding affinity for the antibody is used to form secondary immune complexes, as described above. After washing, the secondary immune complexes may be contacted with a third binding ligand or antibody that has binding affinity for the second antibody, again under effective conditions and for a period of time sufficient to allow the formation of immune complexes (tertiary immune complexes). The third ligand or antibody is typically linked to a detectable label, allowing detection of the tertiary immune complexes thus formed. This system may provide for signal amplification if this is desired.

As detailed above, immunoassays, in their most simple and/or direct sense, are antibody binding assays. Certain preferred immunoassays are the various types of enzyme linked immunosorbent assays (ELISAs) and/or radioimmunoassays (RIA) known in the art.

In one exemplary ELISA, the selective antibodies are immobilized onto a selected surface exhibiting protein affinity, such as a well in a polystyrene microtiter plate. Then, a test composition suspected of containing the antigen, such as a clinical sample, is added to the wells. After binding and/or washing to remove non-specifically bound immune complexes, the bound antigen may be detected. Detection is generally achieved by the addition of another antibody that is linked to a detectable label. This type of ELISA is a simple "sandwich ELISA". Detection may also be achieved by the addition of a second selective antibody, followed by the addition of a third antibody that has binding affinity for the second antibody, with the third antibody being linked to a detectable label.

Another ELISA in which the antigens are immobilized, involves the use of antibody competition in the detection. In this ELISA, labeled antibodies against an antigen are added to the wells, allowed to bind, and/or detected by means of their label. The amount of an antigen in an unknown sample is then determined by mixing the sample with the labeled antibodies against the antigen during incubation with coated wells. The presence of an antigen in the sample acts to reduce the amount of antibody against the antigen available for binding to the well and thus reduces the ultimate signal. This is also appropriate for detecting antibodies against an antigen in an unknown sample, where the unlabeled antibodies bind to the antigen-coated wells and also reduces the amount of antigen available to bind the labeled antibodies.

Irrespective of the format employed, ELISAs have certain features in common, such as coating, incubating and binding, washing to remove non-specifically bound species, and detecting the bound immune complexes. These are described below.

In coating a plate with either antigen or antibody, one will generally incubate the wells of the plate with a solution of the antigen or antibody, either overnight or for a specified period of hours. Where the antigen or antibody is being co-coupled with a neutral material, the antigen or antibody and the neutral material are incubated together to coat the plate. The wells of the plate will then be washed to remove incompletely adsorbed material. Any remaining available surfaces of the wells may then be "coated" with a nonspecific protein that is antigenically neutral with regard to the test antisera.. These include bovine serum albumin (BSA), casein or solutions of milk powder. The coating allows for blocking of nonspecific adsorption sites on the immobilizing surface and thus reduces the background caused by nonspecific binding of antisera onto the surface. If a co-coupling process was performed the same neutral material may be used in the "blocking" step, or a different neutral material may be used.

In ELISAs, it is probably more customary to use a secondary or tertiary detection means rather than a direct procedure. Thus, after binding of a protein or antibody to the well, coating with a non-reactive material to reduce background, and washing to remove unbound material, the immobilizing surface is contacted with the biological sample to be tested under conditions effective to allow immune complex (antigen/antibody) formation. Detection of the immune complex then requires a labeled secondary binding ligand or antibody, and a secondary binding ligand or antibody in conjunction with a labeled tertiary antibody or a third binding ligand.

"Under conditions effective to allow immune complex (antigen/antibody) formation" means that the conditions preferably include diluting the reactants with solutions such as BSA, bovine gamma globulin (BGG) or phosphate buffered saline (PBS)/Tween. These added agents also tend to assist in the reduction of nonspecific background. The "suitable" conditions also mean that the incubation is at a temperature or for a period of time sufficient to allow effective binding. Incubation steps are typically from about 1 to 2 to 4 hours or so, at temperatures preferably on the order of 25°C to 27°C, or may be overnight at about 4°C or so.

Following all incubation steps in an ELISA, the contacted surface is washed so as to remove non-complexed material. A preferred washing procedure includes washing with a solution such as PBS/Tween, or borate buffer. Following the formation of specific immune complexes between the test sample and the originally bound material, and subsequent washing, the occurrence of even minute amounts of immune complexes may be determined.

To provide a detecting means, the second or third antibody will have an associated label to allow detection. Preferably, this will be an enzyme that will generate color development upon incubating with an appropriate chromogenic substrate. Thus, for example, one will desire to contact or incubate the first and second immune complex with a urease, glucose oxidase, alkaline phosphatase or hydrogen peroxidase-conjugated antibody for a period of time and under conditions that favor the development of further immune complex formation (e.g., incubation for 2 hours at room temperature in a PBS-containing solution such as PBS-Tween).

After incubation with the labeled antibody, and subsequent to washing to remove unbound material, the amount of label is quantified, *e.g.*, by incubation with a chromogenic substrate such as urea, or bromocresol purple, or 2,2'-azino-di-(3-ethyl-benzthiazoline-6-sulfonic acid (ABTS), or H₂O₂, in the case of peroxidase as the enzyme label. Quantification is then achieved by measuring the degree of color generated, *e.g.*, using a visible spectra spectrophotometer and relating the value to a similar value produced using a known amount of analyte.

### II. Multiplex Assays

### A. Arrays

The methods described herein may involve the use of arrays. Array technology allows high-throughput screening for gene expression and molecular interactions. Protein array technology is discussed in detail in Pandey and Mann (2000) and MacBeath and Schreiber (2000). These arrays, which typically contain thousands of different proteins or antibodies spotted onto glass slides or immobilized in tiny wells, allow one to examine the biochemical activities and binding profiles of a large number of proteins at once. To examine protein interactions with such an array, a labeled protein may be incubated with each of the target proteins immobilized on the array. The array is then analyzed to determine which of the many proteins the labeled molecule binds, the quantity or concentration of the protein, or other characteristics of the protein. Those of skill in the art are aware of various methods available to analyze the array.

### 1. Protein Biochip Assays

Biochips, in general, comprise a substrate to which an array of capture molecules ("reagents") has been attached, each at a discrete and identifiable location on the substrate surface in such a manner as to be addressable by a detection method of choice. A neutral material may be co-coupled with one or more of the capture molecules on the substrate to reduce the reactivity at those particular locations on the substrate. When the capture molecules are exposed to an analytic sample, analytes in the sample can bind to a capture molecule on the surface for which it has affinity. The capture or interaction between an analyte molecule and a capture molecule is detected or characterized by any of a variety of means. Such detection or characterization methods are known to those of skill in the art, and include but are not limited to detection of fluorescence, luminescence, absorbance, reflectance, transmittance, or refractive index (*e.g.*, surface plasmon resonance, ellipsometry, a resonant mirror method, a diffraction grating coupler waveguide method or interferometry), immunoassays (*e.g.*, ELISA), gas phase ion spectrometry methods, atomic force microscopy or mass spectrometry and, in particular, SELDI. Quantification of the analytes in the sample can be achieved by selecting an appropriate method of detection.

### 2. Bead Arrays

Microsphere based assays may also be analyzed in flow systems or on bead array platforms. In general, bead array platforms image beads and analytes distributed on an array. In this way, imaging of bead arrays is similar to the chips discussed above. However, in contrast to chips where the analyte is identified by its spatial position on the array, bead arrays typically identify the analyte by the encoded microsphere to which it is bound.

For example, Luminex (Austin, TX) describe a method for encoding microspheres according to their fluorescence as taught in Fulton et al, 1997, Clin. Chem. 43:1749-1756 and U.S. Pat. No. 5,736,330. The methodology is based on the principle that fluorescent microspheres (beads) with unique fluorescent profiles can be immobilized to different analyte specific binders and used to create a fluorescence-based array of analyte specific beads where each bead type is specific for a unique analyte. This technology employs a combination of fluorescent dyes that allow each bead to be independently identified. The analyte specific microspheres are mixed together and contacted with a probe(s) that is labeled with a different fluorescent color. The probes bind to their ligands or receptors on the labeled microspheres and are used to determine the specific molecular interaction at the surface of each bead. The samples may be read in a flow cytometer which allows each microsphere to be identified individually and the corresponding probe binding signal to be read.

The microspheres can be covalently coupled to virtually any amine-containing molecule through surface carboxylate groups. Alternatively, avidin-coupled microspheres are available for immobilizing biotinylated molecules (Fulton et al, 1997, Clin. Chem. 43: 1749-1756).

Other examples of commercially available bead arrays include Illumina's BeadXpress™ Reader and BeadStation 500™.

### 3. Antibody Microarrays

An antibody microarray is a specific form of protein microarrays. Antibody microarrays are often used in general research to detect protein expressions from cell lysates and may be used for diagnostic applications, for example for detecting special biomarkers from serum or urine.

### III. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from and scope of the invention.

### EXAMPLE 1- Co-Coupling Procedure

### Concentration of Lutninex Microspheres:

The evaluation of the benefits of mixing active reagents with neutral materials to couple to Luminex Microspheres was done with 50 million Luminex MagPlex® Microspheres. The MagPlex® Microspheres were concentrated to 0.5 mL in 1.5 mL microcentrifuge tubes with a magnetic separation device. In this washing procedure, the MagPlex® Microspheres were pulled to the side of the microcentrifuge tube by the magnetic surface allowing efficient removal of the supernatant from the mcirocentrifuge tubes. The MagPlex® Microspheres in the microcentrifuge tubes were washed twice with Activation Buffer (0.1 M 2-(N-Morpholino)ethanesulfonic acid hemisodium salt, (MES) buffer, pH 6.2).

### Activation of Luminex Microspheres:

A volume of 0.4 mL Activation Buffer was added to each microcentrifuge tube for the activation. Sulfo-NHS (N-Hydroxysulfosuccinimide) and EDC (1-ethyl-3-[3-dimethylaminopropyl]carbodiimide hydrochlorid) were prepared at concentrations of 50 mg/mL. The microspheres were suspended with sonication and vortexing just prior to the addition of volumes of 0.05 mL Sulfo-NHS and EDC (providing 2.5 mg of each) to each microcentrifuge tube containing 50 million MagPlex® Microspheres for activation. The microspheres were suspended again and the microcentrifuge tubes were placed on a rotator to mix the microspheres during the activation reaction. After 20 minutes, the microcentrifuge tubes were removed.

After activation, the activated microspheres are washed twice with Coupling Buffer (0.1 M 2-(N-Morpholino)ethanesulfonic acid hemisodium salt, (MES) buffer, pH 5.0) to remove the excess Sulfo-NHS and EDC. A volume of 1.0 mL Coupling Buffer was added to each microcentrifuge tube containing 50 million microspheres.

### Coupling proteins to Luminex Microspheres:

Proteins were added to the washed MagPlex® Microspheres in Coupling Buffer. As discussed in more detail in the examples below, different amounts of active reagent proteins (Rabbit Polyclonal anti-Chicken IgY Antibody or Purified Chicken IgY for the Poultry Serology Assay; or Mouse Monoclonal anti-Thyroid Stimulating Hormone Antibody for the New Born Assay) were combined with the neutral materials (Purified Rabbit IgG or Purified Bovine IgG) for coupling to the activated MagPlex® Microspheres.

### EXAMPLE 2 - Co-Coupling Rabbit IgG with Chicken IgY or with Rabbit anti-Chicken IgY Improves Function of Internal Controls in Poultry Serology Assay

### Materials:

Luminex MagPlex® Microspheres for various regions
Rabbit anti-Chicken IgY at 2.4 mg/mL - Reagent Coupled to MagPlex® Microspheres
Chicken IgY at 5.7 mg/mL - Reagent Coupled to MagPlex® Microspheres
Purified Rabbit IgG at 5.0 mg/mL - Neutral Material Coupled to MagPlex® Microspheres
Biotinylated Rabbit anti-Chicken IgY Assay Reagent
Streptavidin-R-phycoerythrin Assay Reagent
PBS-BSA Buffer
PBS-Tween, BSA Buffer

### Procedure and Observations:

### Initial Reagent Coupling Requirements for the Poultry Serology Assay Internal Controls:

Rabbit anti-Chicken IgY and Chicken IgY proteins were coupled to MagPlex® Microspheres to become Internal Controls in the Poultry Serology Assay. The microspheres coupled with Rabbit anti-Chicken IgY were used to indicate that the Chicken serum sample, containing IgY, had been added into the assay. The microspheres coupled with Chicken IgY were used to indicate that the detection antibody, biotinylated Rabbit anti-Chicken IgY, had been added into the assay.

10 µg of Rabbit anti-Chicken IgY and 5 µg of the Chicken IgY were coupled to 50 million MagPlex® microspheres. A sample of chicken serum diluted to 1:500 with Sample Diluent (bovine and porcine proteins with ProClin® (2-Methyl-4-isothiazolin-3-one, 5-Chloro-2-methyl-4-isothiazolin-3-one and 1,2-Benzisothiazolin-3-one)) as a preservative) was added to wells in microtiter plate containing the Capture Reagents (Rabbit anti-Chicken IgY or Chicken IgY proteins, which were coupled to microspheres as discussed above). After the incubation of the sample and Capture Reagent, the microtiter plate was washed to remove unreacted sample while retaining the Capture Reagent in the well. The Detection Reagent (Biotinylated Rabbit anti-Chicken IgY) was then added. After the incubation with this Detection Reagent, the microtiter plate was washed to remove unreacted Detection Reagent. The Reporter Reagent was then added. After the incubation with this Reporter Reagent (Streptavidin-R-phycoerythrin), the microtiter plate was washed to remove unreacted Reporter Reagent. Buffer was added to the well and the contents were measured using a Luminex LX200™ Instrument. The signals produced were determined to be in a range of 23,000 to 44,000 MFI equivalents for the Luminex LX200™ Instrument. This was well above the usable range for the LX200™ Instrument, which has a maximum usable signal of about 20,000 MFI.

The two reagents were coupled to 50 million MagPlex® Microspheres at reduced amounts to attempt to reduce the total MFI response in the assay. The quantities of Chicken IgY coupled were 1.68, 1.24, and 0.84 µg Chicken IgY and 2.4, 1.2, 0.6 µg Rabbit anti-Chicken IgY. MFI responses remained above 25,000 MFI for most of the couplings. In some cases reduced signals (15,000 MFI in one case) were achieved, but this lowest signal did not correspond to the microspheres coupled to the lowest amount of reagent. In another case, a reduction was observed, but the results were highly variable ranging from 16,000 to 23,000 MFI. Repeated experiments yielded variable results with coefficients of variance up to 36% for eight replicates. Thus, it was concluded that simply reducing the amount of reagent in the coupling reaction did not provide reliable data and was not effective in reducing high reactivity of reagents.

### Co-Coupling to Prepare Poultry Serology Assay Internal Controls with Reduced Response Range:

Reagents and neutral materials were mixed as described in Groups 1-4 below and coupled to 50 million microspheres as described in Example 1 above.
Group 1 - A mixture of 2.5 µg Rabbit anti-Chicken IgY Antibody and 147.5 µg Purified Rabbit IgG were coupled to the activated MagPlex® Microspheres.
Group 2 - A mixture of 1.25 µg Rabbit anti-Chicken IgY Antibody and 148.75 µg Purified Rabbit IgG were coupled to the activated MagPlex® Microspheres.
Group 3 - A mixture of 2.5 µg Chicken IgY and 147.5 µg Purified Rabbit IgG were coupled to the activated MagPlex® Microspheres.
Group 4 - A mixture of 1.25 µg Chicken IgY and 148.75 µg Purified Rabbit IgG were coupled to the activated MagPlex® Microspheres.

A sample of chicken serum diluted to 1:500 with Sample Diluent was added to wells in microtiter plate containing the Capture Reagents as described in Groups 1 to 4. After the incubation of the sample and Capture Reagent, the microtiter plate was washed to remove unreacted sample while retaining the Capture Reagent in the well. The Detection Reagent (Biotinylated Rabbit anti-Chicken IgY) was then added. After the incubation with this Detection Reagent, the microtiter plate was washed to remove unreacted Detection Reagent. The Reporter Reagent (Streptavidin-R-phycoerythrin) was then added. After the incubation with this Reporter Reagent, the microtiter plate was washed to remove unreacted Reporter Reagent. Buffer was added to the well and the contents were measured using a Luminex LX200™ Instrument

The objective of using the mixtures described above was to reduce the immunoassay response to a value of less than 20,000 MFI with the Luminex LX200™ Instrument. This was achieved in all four groups.
Group 1 - assay response was 17,000 MFI.
Group 2 - assay response was 12,000 MFI.
Group 3 - assay response was 19,400 MFI.
Group 4 - assay response was 13,900 MFI.

Thus, it was concluded that co-coupling of a neutral material (e.g. Rabbit IgG) with active reagents (Rabbit Polyclonal anti-Chicken IgY or Purified Chicken IgY) to the substrate resulted in reliable diminution of reactivity of the active reagents in the assay.

### EXAMPLE 3 - Co-Coupling of Reagent in New Born 4-Plex Assay

A mixture of 300 µg Mouse Monoclonal anti-Thyroid Stimulating Hormone Antibody and 50 µg Purified Bovine IgG were coupled to the activated MagPlex® Microspheres as described in Example 1 above. A volume of extracted blood sample was added to a well in a microtiter plate containing the Capture Reagent (Mouse Monoclonal anti-Thyroid Stimulating Hormone Antibody, which was co-coupled with Purified Bovine IgG to MagPlex® Microspheres as described above). The Detection Reagent (Biotinylated Mouse Monoclonal anti-Thyroid Stimulating Hormone Antibody) was also added to the well. After the incubation of the sample, Capture Reagent, and Detection Reagent, the microtiter plate was washed to remove unreacted sample and Detection Reagent while retaining the Capture Reagent in the well. The Reporter Reagent (Streptavidin-R-phycoerythrin) was then added. After the incubation with this Reporter Reagent, the microtiter plate was washed to remove unreacted Reporter Reagent. Buffer was added to the well and the contents were measured using a Luminex LX200™ Instrument. This resulted in an assay response of 14,000 MFI, which is within a range that is acceptable for use in generating a standard curve for the TSH Assay.

### REFERENCES

The following references, to the extent that they provide exemplary procedural or other details supplementary to those set forth herein, are specifically referenced herein.
U.S. Patent 3,817,837
U.S. Patent 3,850,752
U.S. Patent 3,939,350
U.S. Patent 3,996,345
U.S. Patent 4,275,149
U.S. Patent 4,277,437
U.S. Patent 4,366,241
Bangham et al., J. Mol. Biol., 13(1):238-252; 253-259,1965.
De Jager et al., Semin. Nucl. Med., 23(2):165-179, 1993.
Deamer and Uster, In: Liposome Preparation: Methods and Mechanisms, Ostro (Ed.), Liposomes, 1983.
Doolittle and Ben-Zeev, Methods Mol, Biol,, 109:215-237, 1999.
Freifelder, In: Physical Biochemistry Applications to Biochemistry and Molecular Biology, 2nd Ed. Wm. Freeman and Co., NY, 1982.
Ghosh and Bachhawat, In: Liver Diseases, Targeted Diagnosis and Therapy Using Specific Receptors and Ligands, Wu et al. (Eds.), Marcel Dekker, NY, 87-104, 1991.
Gregoriadis, In: Drug Carriers in Biology and Medicine, Gregoriadis (Ed.), 287-341, 1979. Gulbis and Galand, Hum. Pathol., 24(12):1271-1285, 1993.
MacBeath and Schreiber, Science, 289(5485):1760-1763, 2000.
Nakamura et al., In: Handbook of Experimental Immunology (4th Ed.), Weir et al., (Eds). 1:27, Blackwell Scientific Publ., Oxford, 1987.
Pandey and Mann, Nature, 405(6788):837-846, 2000.
Szoka and Papahadjopoulos, Proc. Natl. Acad. Sci. USA, 75:4194-4198, 1978.

## Claims

1. A method of controlling reactivity of a reagent in a multiplex immunoassay comprising:
(a) identifying a reagent having a high reactivity in a multiplex immunoassay resulting in an assay signal that is above a maximum usable signal for the multiplex immunoassay;
(b) combining the high-reactivity reagent with a neutral material in a composition, wherein the neutral material is a material that is antigenically neutral with regard to the reagent and the sample in the assay;
(c) applying the composition to a substrate under conditions suitable to couple the high-reactivity reagent and the neutral material to the substrate; and
(d) confirming that the assay signal of the high-reactivity reagent co-coupled with the neutral material to the substrate is within a usable signal range for the multiplex immunoassay.

2. The method of claim 1 comprising controlling the reactivity of two or more reagents having high reactivity in the multiplex immunoassay.

3. The method of claim 1, wherein the assay signal is a chemiluminescent signal or a fluorescent signal.

4. The method of claim 1, wherein the high-reactivity reagent is an antibody or an antigen.

5. The method of claim 1, wherein the neutral material is a non-relevant species antibody or a serum albumin.

6. The method of claim 1, wherein the substrate is a particle or a surface of a well.

7. The method of claim 6, wherein the particle is a microsphere.

8. The method of claim 1, wherein the ratio of high-reactivity reagent to neutral material in the composition is between about 1:120 to 6:1.

9. The method of claim 1, wherein the high-reactivity reagent and the neutral material are covalently coupled to the substrate.

## Patentansprüche

1. Verfahren zur Kontrolle der Reaktivität eines Reagens in einem Multiplex-Immunassay, umfassend:
(a) Identifizieren eines Reagens mit einer hohen Reaktivität in einem Multiplex-Immunassay, das ein Testsignal ergibt, das über einem maximalen nutzbaren Signal für den Multiplex-Immunassay liegt;
(b) Kombinieren des Reagens mit hoher Reaktivität mit einem neutralen Material in einer Zusammensetzung, wobei es sich bei dem neutralen Material um ein Material handelt, das in Bezug auf das Reagens und die Probe im Test antigenneutral ist;
(c) Aufbringen der Zusammensetzung auf ein Substrat unter für die Kopplung des Reagens mit hoher Reaktivität und des neutralen Materials an das Substrat geeigneten Bedingungen; und
(d) Bestätigen, dass das Testsignal des gemeinsam mit dem neutralen Material an das Substrat gekoppelten Reagens mit hoher Reaktivität innerhalb eines nutzbaren Signalbereichs für den Multiplex-Immunassay liegt.

2. Verfahren nach Anspruch 1, umfassend Kontrollieren der Reaktivität von zwei oder mehr Reagentien mit hoher Reaktivität im Multiplex-Immunassay.

3. Verfahren nach Anspruch 1, wobei es sich bei dem Testsignal um ein Chemilumineszenzsignal oder ein Fluoreszenzsignal handelt.

4. Verfahren nach Anspruch 1, wobei es sich bei dem Reagens mit hoher Reaktivität um einen Antikörper oder ein Antigen handelt.

5. Verfahren nach Anspruch 1, wobei es sich bei dem neutralen Material um einen Antikörper einer nicht relevanten Spezies oder ein Serumalbumin handelt.

6. Verfahren nach Anspruch 1, wobei es sich bei dem Substrat um ein Partikel oder eine Oberfläche eines Näpfchens (Well) handelt.

7. Verfahren nach Anspruch 6, wobei es sich bei dem Partikel um ein Mikrokügelchen handelt.

8. Verfahren nach Anspruch 1, wobei das Verhältnis von Reagens mit hoher Reaktivität zu neutralem Material in der Zusammensetzung zwischen etwa 1:120 und 6:1 liegt.

9. Verfahren nach Anspruch 1, wobei das Reagens mit hoher Reaktivität und das neutrale Material kovalent an das Substrat gekoppelt sind.

## Revendications

1. Procédé de contrôle de la réactivité d'un réactif dans un immunodosage multiplexé, comprenant les étapes consistant à :
(a) identifier un réactif ayant une réactivité élevée dans un immunodosage multiplexé, conduisant à un signal de dosage qui est au-dessus d'un signal maximum utilisable pour l'immunodosage multiplexé ;
(b) combiner le réactif à haute réactivité avec un matériel neutre dans une composition, dans laquelle le matériel neutre est un matériel qui est neutre d'un point de vue antigénique eu égard au réactif et à l'échantillon dans le dosage ;
(c) appliquer la composition à un substrat dans des conditions appropriées pour coupler au substrat le réactif à haute réactivité et le matériel neutre ; et
(d) confirmer que le signal de dosage du réactif à haute réactivité couplé, conjointement avec le matériel neutre, au substrat se trouve dans une gamme de signaux utilisables pour l'immunodosage multiplexé.

2. Procédé, selon la revendication 1, comprenant le contrôle de la réactivité de deux ou plusieurs réactifs ayant une réactivité élevée dans l'immunodosage multiplexé.

3. Procédé selon la revendication 1, dans lequel le signal de dosage est un signal chimiluminescent ou un signal fluorescent.

4. Procédé selon la revendication 1, dans lequel le réactif à haute réactivité est un anticorps ou un antigène.

5. Procédé selon la revendication 1, dans lequel le matériel neutre est un anticorps d'une espèce non pertinente ou une albumine sérique.

6. Procédé selon la revendication 1, dans lequel le substrat est une particule ou une surface d'un puits.

7. Procédé selon la revendication 6, dans lequel la particule est une microsphère.

8. Procédé selon la revendication 1, dans lequel le rapport du réactif à haute réactivité au matériel neutre dans la composition est compris entre environ 1:120 et 6:1.

9. Procédé selon la revendication 1, dans lequel le réactif à haute réactivité et le matériel neutre sont couplés par covalence au substrat.
